# EUROPÄISCHE PATENTSCHRIFT

(11) **EP 1 824 426 B1**
(45) Veröffentlichungstag und Bekanntmachung des Hinweises auf die Patenterteilung: **23.07.2008**
(21) Anmeldenummer: 05801610.6
(22) Anmeldetag: 08.11.2005
(51) Int. Cl.: A61F 2/42

(54) **SPRUNGGELENKSENDOPROTHESENELEMENTE**
ENDOPROSTHETIC ELEMENTS FOR AN ANKLE JOINT
ELEMENTS D'ENDOPROTHÈSE D'ARTICULATION TIBIOTARSIENNE

(30) Priorität: 08.11.2004 AT 18622004
(43) Veröffentlichungstag der Anmeldung: 29.08.2007
(60) Teilanmeldung: 08001262.8
(73) Patentinhaber: Alphamed Medizintechnik Fischer GmbH, A-8301 Lassnitzhöhe (AT)
(72) Erfinder: FELLINGER, Michael, A-8010 Graz (AT); ORTHNER, Ernst, A-4600 Wels (AT); SIORPAES, Robert Bezirkskrankenhaus St. Johann, A-6380 St. Johann (AT)
(74) Vertreter: Schwarz, Albin
(86) Internationale Anmeldenummer: PCT/AT2005/000443
(87) Internationale Veröffentlichungsnummer: WO 2006/047805

(56) Entgegenhaltungen:
- WO-A-00/69373
- DE-C1- 10 123 124
- US-A- 3 889 300
- US-A- 3 975 778
- LEARDINI A ET AL: "COMPUTER-ASSISTED DESIGN OF THE SAGITTAL SHAPES OF A LIGAMENT-COMPATIBLE TOTAL ANKLE REPLACEMENT" MEDICAL AND BIOLOGICAL ENGINEERING AND COMPUTING, PETER PEREGRINUS LTD. / IFMBE, HERTS, GB, Bd. 39, Nr. 2, März 2001 (2001-03), Seiten 168-175, XP001099761 ISSN: 0140-0118

## Beschreibung

Die vorliegende Erfindung betrifft Sprunggelenksprothesenelemente, genauer eine Taluskomponente und eine Tibiakomponente-Inlay-Kombination für eine mehrteilige Sprunggelenksprothese, sowie aus diesen Elementen bestehende Sprunggelenksprothesen.

Angesichts der vielen Gründe, die für eine bewegliche Lösung eines schmerzhaft destruierten oberen Sprunggelenks sprechen, war es nur folgerichtig, nach den ersten Erfolgen der Hüft- und Kniegelenkendoprothetik nach entsprechenden Lösungen auch für andere Gelenke zu suchen, zumal an den direkt vom Körpergewicht belasteten Gelenken eine erfolgversprechende Alternative im Sinne einer Resektionsarthroplastik nicht zur Verfügung stand.

Bei den ersten Sprunggelenksprothesen handelte es sich um zementierte Metall-Polyethylen-Gelenke. Die bekannteste Prothese dieser Generation, die sogenannte St. Georg-Endoprothese, wies eine schlittenartige Taluskomponente auf, die mit einer nach unten kongruent, d.h. konkav geformten Tibiakomponente artikulierte.

Die zweite Generation der nun auch zementfrei implantierbaren Endoprothesen stützte sich auf die Idee der "meniskalen" Oxford-Knieendoprothesen. Die schlittenartige Form der metallischen Taluskomponente wurde mit einer oberflächlichen Führung für einen kongruent geformten Polyethylengleitkern, der nach oben mit der planaren Unterfläche der Tibiakomponente frei beweglich artikuliert, beibehalten. Hierdurch ist der Gleitkern (Inlay) durch die entsprechend zur Talusrolle kongruent geformte Unterfläche gegen Luxationen gesichert, lässt aber nach oben hin alle Bewegungsrichtungen, u.a. im Rotationssinn und anterodorsal, zu.

Es stellte sich prinzipiell heraus, dass den mechanischen Anforderungen und somit einer hohen Langzeitüberlebensrate nur eine Sprunggelenkprothese mit einem anatomischen und biomechanisch kompatiblen Design genügen kann, die mit minimaler Knochenresektion implantiert und frei von den verbleibenden Bändern und Sehnen geführt wird. In Beachtung dieser Anforderungen sind verschiedene Typen von Sprunggelenkprothesen entwickelt und zur Behandlung eingesetzt worden.

Die heute verwendeten Endoprothesen können im Wesentlichen in ein- und multiaxiale Zweikomponentenendoprothesen sowie Dreikomponentenendoprothesen unterteilt werden.

Die Dreikomponentenendoprothesen bestehen im Allgemeinen aus einer Tibia- und einer Taluskomponente sowie einem dazwischenliegenden Gleitkern.

Bekannt ist z.B. eine Dreikomponenten-Prothese (HINTEGRA^{®}; Newdeal SA, Vienne, Frankreich) mit einer anatomischen Tibia- und Taluskomponente sowie einem freibeweglichen Polyethylen(PE)-Gleitkern von ultra-hoher Dichte (Inlay). Die Tibiakomponente weist eine Metallplatte mit tibialseits kleinen Verankerungspyramiden sowie einem ventralen Schild auf, welcher eine Schraubenfixation durch zwei ovale Löcher erlaubt und das Einwachsen von Narbengewebe und somit Bewegungseinschränkung verhindert. Die Taluskomponente weist eine einem Konus mit einem schmäleren medialen Seitenradius ähnliche Form auf. Ein Seitenrand medial- und lateralseits führt den PE-Gleitkern. Dieser mobile und geführte Gleitkern weist eine flache Oberfläche zur Tibiakomponente und eine konkave Oberfläche zur Taluskomponente auf.

Ein weiteres Beispiel für eine Dreikomponenten-Prothese wird in der deutschen Patentschrift DE 101 23 124 offenbart.

Bei einer anderen bekannten Sprunggelenkendoprothese ("A.E.S- Prothese", Biomet Merck Deutschland GmbH) besteht die Tibiakomponente aus einer Metallplatte mit einem intraoperativ aufsetzbaren Kiel, während die Taluskomponente als Teil einer Zylinderoberfläche konfiguriert ist, die in Umfangsrichtung einen mittigen rinnenförmigen Einschnitt aufweist, in dem der talusseitig entsprechend geformte Inlay gleitet. Die Oberseite des Inlays ist der Tibiakomponente entsprechend planar ausgebildet.

Die Tibiakomponente einer weiteren bekannten Prothese (LINK S.T.A.R.^{®}; Waldemar Link GmbH & Co. KG) besteht aus einer Metallplatte mit hochglanzpolierter flacher Artikulationsfläche, welche zwei rückseitig angeordnete zylindrische Fixationsstege zur Verankerung im Tibiaknochen besitzt. Als talare Komponente dient eine konvexe Metallkappe, die an ihrer Unterseite in einem abgerundeten stumpfen Winkel zusammenläuft. In der Mitte der konvexen Oberfläche der Taluskomponente befindet sich, A/P-verlaufend, eine Rippe zur Führung des PE-Inlays, der dementsprechend eine konkave Oberfläche mit einer Rinne besitzt.

Die Patentschrift US 3,975,778 offenbart ebenfalls eine Prothese mit einer Tibia - und einer Taluskomponente.

Die vorliegende Erfindung stellt sich die Aufgabe, Sprunggelenksprothesenelemente bzw. Sprunggelenksprothesen bereitzustellen, die ein seitliches Überstehen des Gleitkems und somit die Entstehung eines Impingement verhindern.

Außerdem soll ein talarer Prothesenanteil eine optimale Passform bei gleichzeitiger geringer Knochenresektion besitzen.

Ein Aspekt der Erfindung betrifft eine Tibiakomponente-Inlay-Kombination für eine mehrteilige Sprunggelenksprothese, wobei die Tibiakomponente eine Platte und ein Mittel zur Verankerung im Knochen umfasst und das Inlay eine planare tibiale Oberfläche aufweist. Erfindungsgemäß sind an der dem Inlay zugewandten Seite der Platte eine im Wesentlichen zentrale Erhebung und in der tibialen Oberfläche des Inlay für die Erhebung eine Ausnehmung vorgesehen, wobei die Ausnehmung derart gestaltet ist, dass dem Inlay gegenüber der Tibiakomponente eine Rotation sowie ein AP-Gleiten ermöglicht wird und eine seitliche Bewegung des Inlay verhindert wird.

Hierdurch wird ein seitliches Überstehen des Gleitkerns und somit die Entstehung eines Impingement verhindert.

Gemäß der Erfindung ist die Erhebung kugelsegmentförmig ausgebildet und die Ausnehmung als Segment eines Zylinders mit kugelsegmentförmigen Enden ausgestaltet, wobei die Achse des Zylinders in A/P-Richtung ausgerichtet ist.

In der oben beschriebenen Ausführungsform ist die Ausnehmung im Inlay und die in diese Ausnehmung eingreifende Erhebung in der Platte der Tibiakomponente vorgesehen. Gemäß einer weiteren bevorzugten Ausführungsform ist es gerade umgekehrt: Diese betrifft daher eine Tibiakomponente-Inlay-Kombination für eine mehrteilige Sprunggelenksprothese, wobei die Tibiakomponente eine Platte und ein Mittel zur Verankerung im Knochen umfasst und das Inlay eine planare tibiale Oberfläche aufweist, wobei an der dem Inlay zugewandten Seite der Platte eine im Wesentlichen zentrale Ausnehmung und in der tibialen Oberfläche des Inlay für diese Ausnehmung eine Erhebung vorgesehen sind. Die Ausnehmung ist derart gestaltet, dass dem Inlay gegenüber der Tibiakomponente eine Rotation sowie ein AP-Gleiten ermöglicht wird und eine seitliche Bewegung des Inlay verhindert wird. Die Erhebung ist kugelsegmentförmig ausgebildet und ist die Ausnehmung als Segment eines Zylinders mit kugelsegmentförmigen Enden ausgestaltet, wobei die Achse des Zylinders in A/P-Richtung ausgerichtet ist.

Vorzugsweise umfasst das Mittel zur Verankerung im Knochen einen ventral gebogenen Keil.

Ein weiterer Erfindungsgegenstand betrifft eine dreiteilige Sprunggelenksprothese, welche die erfindungsgemäße Tibiakomponente-Inlay-Kombination und eine Taluskomponente umfasst.

Die Taluskomponente weist eine Unterseite auf, die nach Implantation dem knöchernen Talus zugewandt ist, wobei die dem knöchernen Talus zugewandte Unterseite als konkaves Kugelsegment ausgestaltet ist.

Es hat sich gezeigt, daß die konkave Ausgestaltung der Unterseite der Taluskomponente eine besonders schonende Präparation des Knochens ermöglicht.

Es kann an der als konkaves Kugelsegment ausgestalteten Unterseite ein Mittel zur Verankerung im Knochen vorgesehen werden.

Vorzugsweise umfasst das Mittel einen zentral angeordneten rohrförmigen Stiel und einen dazu ventral angeordneten Stift.

Nachfolgend wird die Erfindung anhand der Zeichnung näher erläutert, wobei Fig. 1 und 2 einen Querschnitt bzw. eine Ansicht einer Ausführungsform der Taluskomponente, Fig. 3, 4 und 5 Ansichten einer Ausführungsform der Tibiakomponente einer erfindungsgemäßen Tibiakomponente-Inlay-Kombination und Fig. 6, 7 und 8 eine Ansicht bzw. Querschnitte einer Ausführungsform des Inlays einer erfindungsgemäßen Tibiakomponente-Inlay-Kombination veranschaulichen. Die Fig. 9 und 10 zeigen eine weitere Ausführungsform einer Tibiakomponente bzw. eines Inlays.

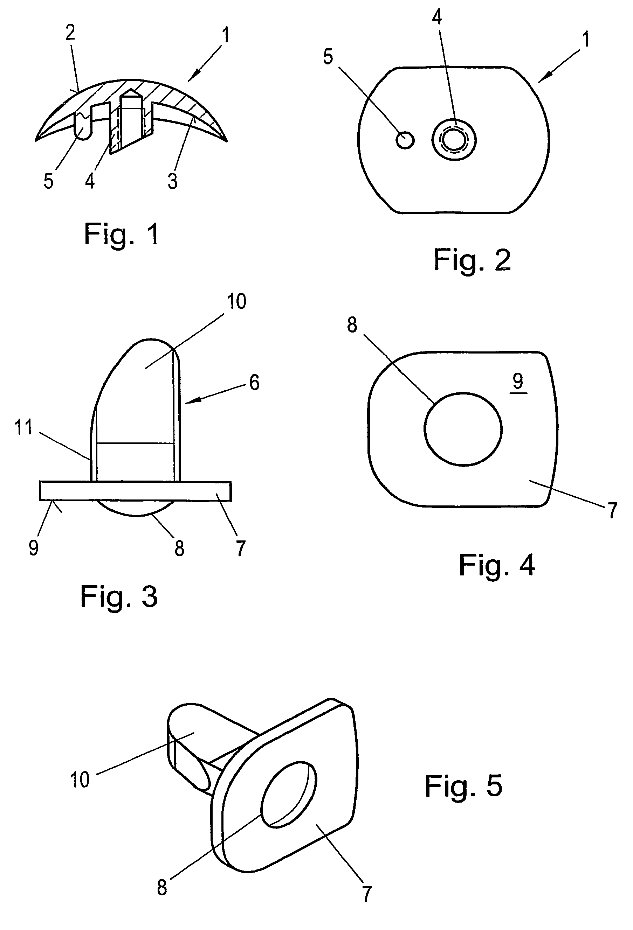

Fig. 1 zeigt einen Schnitt durch eine Ausführungsform der Taluskomponente 1 gemäß Anspruch 4. Dieses Element ist gewöhnlich aus biokompatiblem Metall gefertigt, beispielsweise aus CoCrMo nach ISO 5832/4. Bezugsziffer 2 bezeichnet die dem Inlay zugewandte zylindrisch konvexe Oberfläche. Mit 3 ist die als konkaves Kugelsegment ausgestaltete, dem Talus zugewandte Unterseite des Elements bezeichnet, die zum Beispiel mit Tricalciumphosphat beschichtet sein kann.

Fig. 2 zeigt eine Ansicht der Unterseite 3 der Taluskomponente 1. Im zentralen Teil der Unterseite 3 ist ein rohrförmiger Stiel 4 angeordnet, der zur Verankerung im Knochen dient und, wie dargestellt, mit einem Innengewinde versehen sein kann. Dieser ist in Richtung des zentralen Radials der sphärischen Oberfläche ausgerichtet. Um das Einbringen des Gelenksteils zu erleichtern, ist der rohrförmige Stiel 4 abgeschrägt, und zwar derart, dass sich der längere Teil vorne befindet.

Ventral des rohrförmigen Stiels 4 ist ein Metallstift 5 angeformt. Dieser hat beispielsweise einen Durchmesser von etwa 3 mm und ist in der Darstellung parallel zur Richtung des zentralen Stiels 4 angeordnet. Der Stift 5 dient zur primären Rotationssicherung des eingebrachten talaren Prothesenelements.

In Fig. 3 ist eine Seitenansicht einer Ausführungsform der Tibiakomponente 6 einer erfindungsgemäßen Tibiakomponente-Inlay-Kombination dargestellt. Das tibiale Prothesenelement besteht aus einer z.B. 3 mm dicken Metallplatte 7, welche auf der Seite des Inlays eine kugelsegmentförmige Erhebung 8 trägt, welche einen Teil für die notwendige Zwangsführung des Polyinlays bildet. Die Erhebung 8 ist im Wesentlichen zentral an der Unterseite 9 der Metallplatte 7 vorgesehen, wie Fig. 4 und 5 zeigen.

Zur Vergrößerung der ossären Integrationsfläche sowie zur Rotationssicherung der Tibiakomponente 6 ist diese kranialwärts mit einem Keil 10 versehen. Dieser Keil 10 ist an seiner unteren Begrenzungsseite 11 deutlich abgeflacht, um die Insertion der Tibiakomponente 6 zu erleichtern. Der kraniale Keilanteil verjüngt sich kontinuierlich und soll damit zur Impaktierung des spongiösen Knochen beitragen.

Vorzugsweise können individuell auf die entsprechende Metallplatte 7 verschieden lange Keile je nach Erfordernis aufgesetzt werden. Der längste Revisionskeil trägt dabei bevorzugt 2 Löcher für die Aufnahme von Verriegelungsbolzen. In die kugelsegmentförmige Erhebung 8 sowie in die Metallplatte 7 kann ein Befestigungsmechanismus für die modularen Tibiakeile integriert werden.

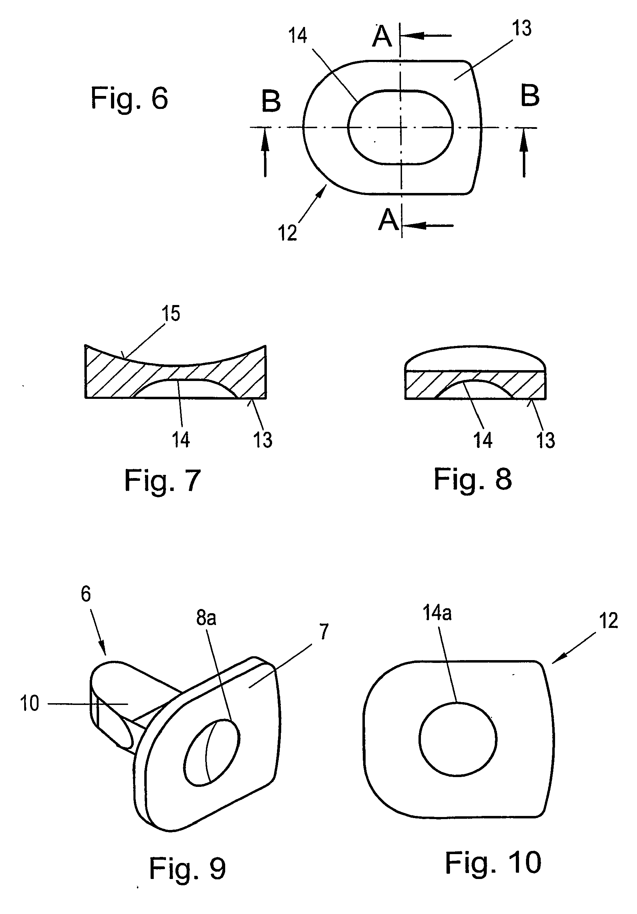

Fig. 6 zeigt eine Ansicht der tibialen Oberfläche 13 des PE-Inlays 12, der beispielsweise aus Chirulen ISO 5834 gefertigt sein kann. Die Konfiguration des Inlays 12 orientiert sich bezüglich der Form an der Metallplatte 7 der Tibiakomponente 6. Die zu verwendende Größe des Inlay 12 ist prinzipiell an der Taluskomponente angelehnt.

Die tibiale Oberfläche 13 besteht aus einer planaren Fläche, in deren Zentrum in Längsrichtung, d.h. A/R-Richtung, eine Ausnehmung 14 in Form einer Rinne vorgesehen ist, die als Zylindersegment mit kugelsegmentförmigen Enden ausgestaltet ist. Diese Form der Ausnehmung 14 lässt sich aus den in Fig. 7 und 8 dargestellten Querschnitten entlang der Linie B-B bzw. A-A der Fig. 6 erkennen. Die Ausnehmung 14 nimmt die Erhebung 8 der Tibiakomponente 6 geschlossen auf. Im Zusammenspiel ist somit das notwendige A/P-Gleiten und die Rotation des Inlays 12 gegenüber der Tibiakomponente 6 gewährleistet. Die seitliche Verschiebbarkeit gegenüber der Tibiakomponente 6 ist hingegen eingeschränkt. Dies verhindert in Bezug auf die Metallplatte 7 der Tibiakomponente 6 ein seitliches Überstehen und somit die Entstehung eines Impingements durch den Inlay 12.

Die der Taluskomponente 1 zugewandte Oberfläche 15 des Inlays 12 ist, wie in Fig. 7 dargestellt, dieser entsprechend als konkave Zylinderoberfläche ausgebildet.

Mit den Fig. 9 und 10 ist die umgekehrte Ausführungsform gezeigt, bei welcher die Ausnehmung nicht im Inlay, sondern in der Tibiakomponente vorgesehen ist. In der Fig. 9 ist die Ausnehmung mit dem Bezugszeichen 8a in der Platte 7 der Tibiakomponente gezeigt. Das Gegenstück, also die Erhebung, welche in die Ausnehmung eingreift, ist dann im Inlay 12 vorgesehen, wie mit dem Bezugszeichen 14a in der Fig. 10 gezeigt ist.

Die Ausnehmung 8a in der Platte 7 kann auch rinnenförmig ausgebildet sein, analog der rinnenförmigen Ausnehmung 14 des Inlays 12 (Fig. 6), und zwar auch in Längsrichtung, d.h. A/R-Richtung. In diesem Fall ist die Ausnehmung 8a dann als Zylindersegment mit kugelsegmentförmigen Enden ausgestaltet.

## Patentansprüche

1. Tibiakomponente-Inlay-Kombination für eine mehrteilige Sprunggelenksprothese, wobei die Tibiakomponente (6) eine Platte (7) und ein Mittel zur Verankerung im Knochen umfasst und das Inlay (12) eine planare tibiale Oberfläche aufweist, wobei an der dem Inlay (12) zugewandten Seite der Platte (7) eine im Wesentlichen zentrale Erhebung (8) und in der tibialen Oberfläche des Inlay (12) für die Erhebung (8) eine Ausnehmung (14) vorgesehen sind, und die Ausnehmung (14) derart gestaltet ist, dass dem Inlay (12) gegenüber der Tibiakomponente (6) eine Rotation sowie ein AP-Gleiten ermöglicht wird und eine seitliche Bewegung des Inlay (12) verhindert wird,
**dadurch gekennzeichnet,**
**dass** die Erhebung (8) kugelsegmentförmig ausgebildet und die Ausnehmung (14) als Segment eines Zylinders mit einer Achse und mit kugelsegmentförmigen Enden ausgestaltet ist, wobei die Achse des Zylinders in A/P-Richtung ausgerichtet ist.

2. Tibiakomponente-Inlay-Kombination für eine mehrteilige Sprunggelenksprothese, wobei die Tibiakomponente (6) eine Platte (7) und ein Mittel zur Verankerung im Knochen umfasst und das Inlay (12) eine planare tibiale Oberfläche aufweist, wobei
an der dem Inlay (12) zugewandten Seite der Platte (7) eine im Wesentlichen zentrale Ausnehmung (8a) und in der tibialen Oberfläche des Inlay (12) für diese Ausnehmung (8a) eine Erhebung (14a) vorgesehen sind, wobei die Ausnehmung (8a) derart gestaltet ist, dass dem Inlay (12) gegenüber der Tibiakomponente (6) eine Rotation sowie ein AP-Gleiten ermöglicht wird und eine seitliche Bewegung des Inlay (12) verhindert wird,
**dadurch gekennzeichnet,**
**dass** die Erhebung (14a) kugelsegmentförmig ausgebildet und die Ausnehmung (8a) als Segment eines Zylinders mit einer Achse und mit kugelsegmentförmigen Enden ausgestaltet ist, wobei die Achse des Zylinders in A/P-Richtung ausgerichtet ist.

3. Tibiakomponente-Inlay-Kombination nach einem der Ansprüche 1 bis 2, **dadurch gekennzeichnet, dass** das Mittel zur Verankerung im Knochen einen ventral gebogenen Keil (10) umfasst.

4. Dreiteilige Sprunggelenksprothese mit einer Taluskomponente, einem Inlay und einer Tibiakomponente, **dadurch gekennzeichnet, dass** als Tibiakomponente und Inlay eine Kombination nach einem der Ansprüche 1 bis 3 vorgesehen ist und dass die Taluskomponente eine Unterseite (3) aufweist, die nach Implantation dem knöchernen Talus zugewandt ist und die dem knöchernen Talus zugewandte Unterseite (3) als konkaves Kugelsegment ausgestaltet ist.

## Claims

1. A tibia component-inlay combination for a multipart ankle joint prosthesis, wherein the tibia component (6) comprises a plate (7) and a means for anchoring in the bone and the inlay (12) has a planar tibial surface, wherein a substantially central elevation (8) is provided on the side of the plate (7) facing the inlay (12) and a recess (14) for the elevation (8) is provided on the tibial surface of the inlay (12), with the recess (14) being designed such that the inlay (12) is allowed to rotate and slide in an AP-direction relative to the tibia component (6) and a lateral movement of the inlay (12) is prevented,
**characterized in that**
the elevation (8) is in the shape of a spherical segment and the recess (14) is configured as a segment of a cylinder having an axis and spherical-segment-shaped ends, with the axis of the cylinder being oriented in the A/P-direction.

2. A tibia component-inlay combination for a multipart ankle joint prosthesis, wherein the tibia component (6) comprises a plate (7) and a means for anchoring in the bone and the inlay (12) has a planar tibial surface, wherein a substantially central recess (8a) is provided on the side of the plate (7) facing the inlay (12) and an elevation (14a) for said recess (8a) is provided on the tibial surface of the inlay (12), with the recess (8a) being designed such that the inlay (12) is allowed to rotate and slide in an AP-direction relative to the tibia component (6) and a lateral movement of the inlay (12) is prevented,
**characterized in that**
the elevation (14a) is in the shape of a spherical segment and the recess (8a) is configured as a segment of a cylinder having an axis and spherical-segment-shaped ends, with the axis of the cylinder being oriented in the A/P-direction.

3. A tibia component-inlay combination according to any of claims 1 to 2, **characterized in that** the means for anchoring in the bone comprises a ventrally curved wedge (10).

4. A three-part ankle joint prosthesis comprising a talus component, an inlay and a tibia component, **characterized in that** a combination according to any of claims 1 to 3 is provided as the tibia component and the inlay and that the talus component has a bottom side (3) facing the osseous talus after implantation and that the bottom side (3) facing the osseous talus is configured as a concave spherical segment.

## Revendications

1. Combinaison inlay/composant tibial pour une prothèse d'articulation de la cheville en plusieurs parties, dans laquelle le composant tibial (6) comprend une plaque (7) et un moyen pour l'ancrage dans un os, et l'inlay (12) présente une surface tibiale plane, dans lequel un bossage sensiblement central (8) est prévu sur le côté de la plaque (7) tourné vers l'inlay (12) et un évidement (14) est prévu dans la surface tibiale de l'inlay (12) pour le bossage (8), et ledit évidement (14) est réalisé de manière à permettre à l'inlay (12) une rotation ainsi qu'un glissement antéropostérieur par rapport au composant tibial (6), et à empêcher un mouvement latéral de l'inlay (12),
**caractérisée en ce que** le bossage (8) est réalisé sous la forme d'une calotte sphérique et l'évidement (14) est réalisé sous forme d'un segment de cylindre avec un axe et avec des extrémités en forme de calotte sphérique, l'axe du cylindre étant orienté dans la direction antéropostérieure.

2. Combinaison inlay/composant tibial pour une prothèse d'articulation de la cheville en plusieurs parties, dans laquelle le composant tibial (6) comprend une plaque (7) et un moyen pour l'ancrage dans un os, et l'inlay (12) présente une surface tibiale plane, dans lequel un évidement sensiblement central (8a) est prévu sur le côté de la plaque (7) tourné vers l'inlay (12), et un bossage (14a) est prévu dans la surface tibiale de l'inlay (12) pour cet évidement (8a), et ledit évidement (8a) est réalisé de manière à permettre à l'inlay (12) une rotation ainsi qu'un glissement antéropostérieur par rapport au composant tibial (6), et à empêcher un mouvement latéral de l'inlay (12),
**caractérisée en ce que** le bossage (14a) est réalisé sous la forme d'une calotte sphérique et l'évidement (8a) est réalisé sous la forme d'un segment de cylindre avec un axe et avec des extrémités en forme de calotte sphérique, l'axe du cylindre étant orienté dans la direction antéropostérieure.

3. Combinaison inlay/composant tibial selon l'une des revendications 1 et 2, **caractérisée en ce que** le moyen d'ancrage dans l'os comprend un coin (10) cintré en direction ventrale.

4. Prothèse d'articulation de cheville en trois parties, comprenant un composant de talus, un inlay et un composant tibial, **caractérisée en ce que** l'on prévoit à titre de composant tibial et d'inlay une combinaison selon l'une des revendications 1 à 3, et **en ce que** le composant de talus présente une face inférieure (3) qui après implantation est tournée vers le talus osseux et ladite face inférieure (3) tournée vers le talus osseux est réalisée sous forme de segment sphérique concave.
